# EUROPÄISCHE PATENTSCHRIFT

(11) **EP 4 151 175 B1**
(45) Veröffentlichungstag und Bekanntmachung des Hinweises auf die Patenterteilung: **25.10.2023**
(21) Anmeldenummer: 21197991.9
(22) Anmeldetag: 21.09.2021
(51) Int. Cl.: A61C 5/90

(54) **LIPPEN/WANGEN-HALTER SOWIE SYSTEM AUS INTRAORALSCANNER UND LIPPEN/WANGEN-HALTER**
LIP/CHEEK HOLDER AND SYSTEM COMPRISING INTRA-ORAL SCANNER AND LIP/CHEEK HOLDER
ÉCARTEUR POUR LÈVRES ET JOUES, AINSI QUE SYSTÈME COMPOSÉ D'UN SCANNER INTRABUCCAL ET D'UN ÉCARTEUR POUR LÈVRES ET JOUES

(43) Veröffentlichungstag der Anmeldung: 22.03.2023
(73) Patentinhaber: Ivoclar Vivadent AG, 9494 Schaan (LI)
(72) Erfinder: LICHTENSTEIGER, Markus, 9462 Montlingen (CH); WACHTER, Wolfgang, 9494 Schaan (LI); ENGGIST, Lukas, 7320 Sargans (CH); MÜLLER, Frank, 6800 Feldkirch (AT)
(74) Vertreter: Baldus, Oliver

(56) Entgegenhaltungen:
- EP-A1- 1 709 935
- WO-A1-03/051185

## Beschreibung

Die Erfindung betrifft einen Lippen/Wangen-Halter gemäß dem Oberbegriff von Anspruch 1 sowie ein System aus einem Intraoralscanner und einem Lippen/Wangen-Halter, gemäß dem Oberbegriff von Anspruch 16.

Lippen/Wangen-Halter sind seit langen bekannt und haben sich unter der Marke "Optragate" der Firma Ivoclar Vivadent AG am Markt durchgesetzt.

Derartige Lippen/Wangen-Halter weisen einen Lippenring auf, der sich an den Lippen anliegend um die Mundöffnung des Patienten erstreckt, und einen Vestibulärring, der sich im Vestibulum des Patienten im Wesentlichen ringförmig erstreckt. Diese Ringe sind über eine elastische Folie miteinander verbunden.

Die Lippe des Patienten wird hierdurch abgedeckt und geschützt. Der Zahnarzt kann im Mundinnenraum des Patienten die notwendigen Behandlungsschritte vornehmen, ohne dass die Gefahr gegenseitiger Infektionen durch Schleimhautkontakte bestünde.

Im Rahmen der Behandlungen führt der Zahnarzt regelmäßig Instrumente in den Mundinnenraum ein. Diese Instrumente haben meist Kontakt mit der Folie des Lippen/Wangen-Halters, und zwar sowohl beim Einführen als auch manchmal beim Entfernen. Die Instrumente haben meist einen metallischen Schaft und gleiten mit diesem an der Folie entlang.

Ein Saugschlauch eines Speichelsaugers wird beispielsweise über die Lippe und damit über die Folie des Lippen Wangen Halters gehängt und liegt dort kontinuierlich an.

Manchmal neigen Patienten dazu, während der Behandlung schlucken zu wollen und schließen hierbei unwillkürlich den Mund. Hierdurch entsteht eine Anlage zwischen dem Schaft des Instruments und sowohl der Unterlippe als auch der Oberlippe, genauer gesagt, je der dortigen Folie. Die Folie soll natürlich auch unter diesem Anlagedruck in ihrer Funktion erhalten bleiben und insbesondere nicht an dem Schaft des Instruments anhaften.

Daher ist es aus der DE 10 2005 015 406 B4 bekannt geworden, eine recht gut gleitfähige Folie zu verwenden. Jedoch stellt sich bei der mit dieser Folie vorgenommenen Erprobung ein sogenannter "Stick-Slip-Effekt" ein, der die Scanergebnisse verfälschte.

Die Verwendung einer recht gut gleitfähigen Folie stellt einen Kompromiss dar. Zum einen soll die Folie so dünn wie möglich sein, um sich geschmeidig an den Mund des Patienten anpassen zu können. Zum anderen soll sie reißfest sein.

Das Material der Wahl für die Folie ist typischerweise ein Elastomer. Dieses ist hochelastisch, hat aber gummiartige Eigenschaften. Bei der Einführung eines Intraoralscanners in den Mund des Patienten, der typischerweise einen Kunststoffschaft aufweist, entsteht leicht der sogenannte Stick-Slip-Effekt. Dieser führt dazu, dass der Scanner kurz an der Folie hängen bleibt und sich dann bei der Weiterführung der Bewegung des Scanners wieder von der Folie löst.

Für eine 3D-Aufnahme des Zahnbogens des Patienten ist es aber notwendig, dass der Scanner mit gleichbleibender Geschwindigkeit um den Zahnbogen geführt wird. Bleibt er an der Folie hängen, wird der Fluss der Bewegung unterbrochen, und der Scanprozess kann abreissen. Auch wird die Aufmerksamkeit des Zahnarztes vom eigentlichen Erfassen der 3D-Aufnahmen abgelenkt. Die Führungskraft muss vergrößert werden, um den Scanner erneut in Bewegung zu setzen. Typischerweise entsteht eine eher ruckartige Bewegung.

Die Probleme vergrößern sich noch, wenn der Patient aufgrund des Fremdkörpereffekts den Mund zu schließen versucht, da dann der Anpressdruck zwischen dem Schaft und der Folie erhöht wird.

Der Zahnarzt könnte sich behelfen, indem er die Folie und oder den Schaft mit einem Gleitmittel einreibt. Ein solches Vorgehen läuft jedoch dem Vorteil der Anwendung entgegen, mit Lippen/Wangenhalter ein anwenderfreundliches und simples Accessoire ohne zusätzliche Vorberitung zur Verfügung zu stellen

Demgegenüber liegt der Erfindung die Aufgabe zugrunde, einen Lippen/Wangen-Halter gemäß dem Oberbegriff von Anspruch 1 und ein System aus einem Intraoralscanner und einem Lippen/Wangen-Halter gemäß dem Oberbegriff von Anspruch 16 zu schaffen, der für einen störungsfreien Arbeitsablauf in der Zahnarztpraxis besser geeignet ist.

Diese Aufgabe wird erfindungsgemäß durch die Ansprüche 1 und 16 gelöst. Vorteilhafte Weiterbildungen ergeben sich aus den Unteransprüchen.

Erfindungsgemäß ist es vorgesehen dann, die Folie des Lippen/Wangen-Halters mindestens an den relevanten Stellen, also an den Stellen, an denen eine Anlage zu einem Schaft entstehen kann, mit einem durch eine Behandlung der betreffenden Oberfläche der Folie erzeugten Gleitelement zu versehen.

Die Folie wird hierdurch nicht oder zumindest nicht relevant dicker, sodass sie in ihrer Geschmeidigkeit erhalten bleibt. Das Gleitelement kann entweder eine in atomarer Abscheidung erzeugte Beschichtung oder eine Oberflächenmodifikation sein. Es hat einen wesentlich geringeren Gleitreibungskoeffizient als ein typisches gummiartiges Elastomer. Dieser kann beispielsweise 1,5 oder 1,0 betragen.

Das Gleitelement ist deutlich härter und dementsprechend auch spröder als der Folienkörper im Übrigen. Überraschend haben Versuche ergeben, dass in der Praxis denn noch eine mindestens gleich gute Sicherheit der Folie des Lippen/Wangen-Halter gegen ein Reißen besteht. Dies liegt wohl darin begründet, dass der Stick-Slip-Effekt mit Sicherheit vermieden wird und daher Kräfte, denen die Folie bei der Handhabung ausgesetzt ist, signifikant vermindert sind.

Dies gilt insbesondere für den bukkalen Bereich des Lippen/Wangen-Halter. Dort ist der Schaft des Lippen/Wangen-Halters typischerweise an drei Seiten in Anlage mit der Folie, und daher der Anlagedruck am größten. Dennoch ist auch hier erfindungsgemäß die gleichmäßige Gleitbewegung sichergestellt.

Das Gleitelement kann auch durch eine Kombination aus atomarer Abscheidung und Oberflächenmodifikation, oder nur durch atomare Abscheidung, erzeugt werden. Bei einer Oberflächenbeschichtung mit Siliziumdioxid lässt sich zugleich eine Oberflächenmodifikation realisieren.

Erfindungsgemäß bestehen keine losen Werkstoffe wie bei einer Gleitmittel-Beschichtung. Eine sterile Arbeitsumgebung ist allzeit gewährleistet.

Die erfindungsgemäße Bereitstellung eines besonders niedrigen Gleitreibungskoeffizienten liegt gegenüber allen in der Praxis vorkommenden Materialien von Schäften vor. Ein dentaler Spiegel als dentales Instrument hat typischerweise einen metallischen Schaft. Ein Intraoralscanner hat typischerweise einen auswechselbaren Scankopf. Dieser kann beispielsweise aus Polyurethan oder besser aus autoklavierbarem Polysulfon bestehen. Gegenüber diesen Materialien ist der Gleitreibungskoeffizient erfindungsgemäß geringer als 2.

Das erfindungsgemäße Gleitelement erstreckt sich in vorteilhafter Ausgestaltung nur auf der Innenseite des im Ausgangszustand im Wesentlichen schlauchförmigen Lippen/Wangen-Halters. Entweder ist die Innenseite vollständig mit dem Gleitelement versehen. Oder nur der mittlere Bereich der Innenseite, also der Bereich, der von beiden Ringen beabstandet ist und am Lippenübergang zur Mundhöhle aufliegt, ist mit den Gleitelement versehen. Diese Lösung hat den Vorteil, dass es nicht zur Anlage zwischen dem Gleitelement und der Schleimhaut des Patienten kommt, auch nicht im Vestibulum, da angrenzend an den Vestibulärring die Folie dann kein Gleitelement aufweist. Es ist auch möglich, das Gleitelement nur angrenzend an den Vestibulärring wegzulassen, also beispielsweise nur über den 20 oder 30% der Länge des Lippen/Wangen-Halters, die an den Vestibulärring angrenzen.

Weitere Vorteile, Einzelheiten und Merkmale ergeben sich aus der nachfolgenden Beschreibung mehrerer Ausführungsbeispiele der Erfindung anhand der Zeichnung.

Es zeigen:
- Fig. 1: eine schematische Ansicht eines Systems aus einem Intraoralscanner und einem Lippen/Wangen-Halter in einer ersten erfindungsgemäßen Ausführungsform;
- Fig. 2: eine vergrößerte Darstellung der Ausführungsform gemäß Fig. 1;
- Fig. 3: eine Ausschnittvergrößerung eines Details aus Fig. 2, jedoch unter Darstellung einer anderen Ausführungsform der Erfindung;
- Fig. 4: eine Darstellung gemäß Fig. 3, jedoch in einer dritten erfindungsgemäßen Ausführungsform der Erfindung; und
- Fig. 5: eine Darstellung gemäß Fig. 3, jedoch in einer vierten erfindungsgemäßen Ausführungsform der Erfindung.

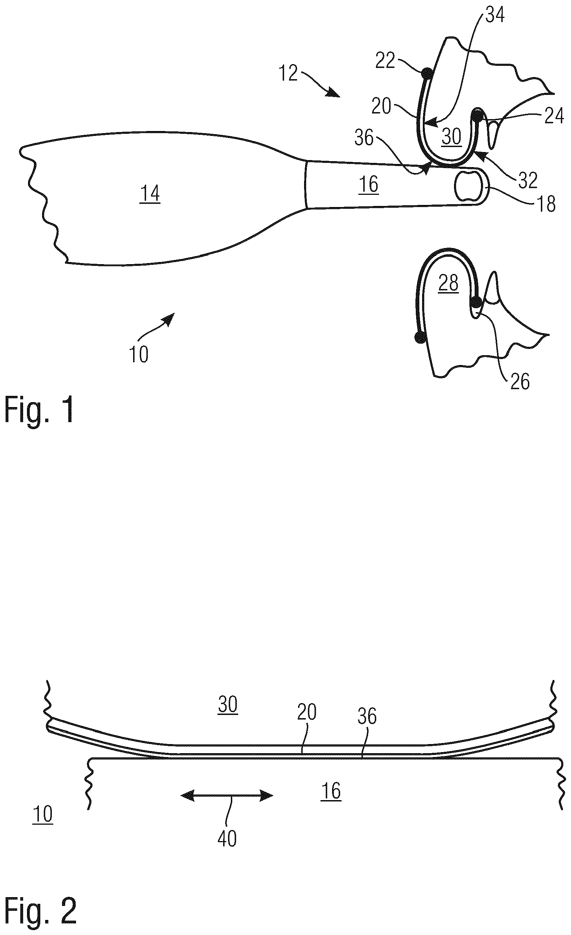

In Fig. 1 ist ein Intraoralscanner 10 und ein Lippen/Wangen-Halter 12 dargestellt. Der Intraoralscanner 10 weist eine Greifhandhabe 14 und einen Schaft 16 auf. Der Schaft 16 ist mit dem Scankopf 18 zusammen abnehmbar und an der Greifhandhabe 14 lösbar befestigt. Der Durchmesser des Schafts 16 beträgt im dargestellten Ausführungsbeispiel 20mm. Der Scanner 10 wird für den scan am Zahnbogen entlang geführt. Er wird in das Vestibulum 26 eingeführt. Hierzu muss die Oberlippe 30 - und bei dem betreffenden Scan natürlich auch die Unterlippe 28 - eingedrückt werden, um mit dem Scankopf 18 auf die Höhe des betreffenden Zahnbogens zu gelangen. Dies bedingt einen entsprechenden Anpressdruck.

Der erfindungsgemäße Lippen/Wangen-Halter 12 besteht aus einer Folie 20 und einen Lippenring 22 und einen Vestibulärring 24. Die Folie 20 erstreckt sich zwischen dem Lippenring 22 und dem Vestibulärring 24. Damit bildet die Folie 20 zwischen den Ringen einen schlauchförmigen Abschnitt aus. Der Vestibulärring 24 hat einen etwas geringeren Durchmesser als der Lippenring 22 und ist dafür bestimmt, im Vestibulum 26 eines Patienten eingelegt zu werden. In aufgespanntem Zustand des Lippen/Wangen-Halter 12 weist dieser zwischen dem Lippenring 22 und dem Vestibulärring 24 eine Einschnürung auf.

Die Folie 20 bedeckt in an sich bekannter Weise die Unterlippe 28 und die Oberlippe 30 des Patienten und verhindert die direkte Anlage zwischen den Lippen 28 und 30 und dem Schaft 16, und zwar auch dann, wenn der Patient seine Lippen um den Schaft 16 schließt.

Die Folie 20 hat eine Innenseite 32 und eine Außenseite 34. Die Innenseite 32 ist die Seite, die zum Schaft 16 hin weist, und die Außenseite 34 ist die Seite, die zu den Lippen 28 und 30 weist.

Erfindungsgemäß ist die Innenseite 32 der Folie 20 mit einem Gleitelement 36 versehen. Das Gleitelement 36 ist durch eine Oberflächenbehandlung der Folie 20, genauer gesagt der Innenseite 32 der Folie, erzeugt worden und weist einen geringen Gleitreibungskoeffizienten von 1 auf.

Das Gleitelement 36 erstreckt sich über die Innenseite 32 des schlauchförmigen Abschnitts der Folie 20 lediglich im Bereich der Einschnürung, also von den Ringen beabstandet.

Die Folie 20 hat in dem dargestellten Ausführungsbeispiel eine Stärke von 0,2 mm. Das Gleitelement 36 hat eine Stärke im atomaren Bereich oder etwas über dem atomaren Bereich. Das Gleitelement 36 wird so erzeugt, dass es mit der Folie fest verankert ist. Im dargestellten Ausführungsbeispiel gemäß den Figuren 1 und 2 besteht es aus Siliciumdioxid-Partikeln, die mindestens teilweise in die Oberfläche der Folie 20 eingebettet sind. Diese besondere Art der Oberflächenbeschichtung ermöglicht es, dass sich auch bei hohen Scherbelastungen keine Elemente des Gleitelements 36 von der Folie 20 lösen können.

Aus Fig. 2 ist eine etwas vergrößerte Ansicht des Anlagebereichs an der eingekreisten Stelle gemäß Fig. 1 zwischen dem Gleitelement 36 und dem Schaft 16 ersichtlich. Die Folie 20 mit dem Gleitelement 36 wird durch die Anlage an dem Schaft 16 in eine gerade Form gebracht, und die Lippe 30 wird entsprechend verformt. In Fig. 2 ist die Dicke des Gleitelement 36 übertrieben dargestellt, damit das Gleitelement 36 überhaupt dargestellt werden kann. Eine Relativbewegung zwischen dem Intraoralscanner 10 und der Lippe 30 ist erfindungsgemäß mit ganz geringer Kraft möglich, auch wenn ein typischer Anpressdruck von 1 bis 10 Newton besteht.

Mit dem erfindungsgemäßen Gleitelement 36 ist sichergestellt, dass kein Stick-Slip-Effekt entsteht, vielmehr erfolgt bei der Bewegung in der Richtung 40 ein gleichmäßiges Gleiten zwischen dem Schaft 16 und dem Lippen/Wangen-Halter 12. Der Unterschied zwischen Haftreibung und Gleitreibung ist entsprechend gering.

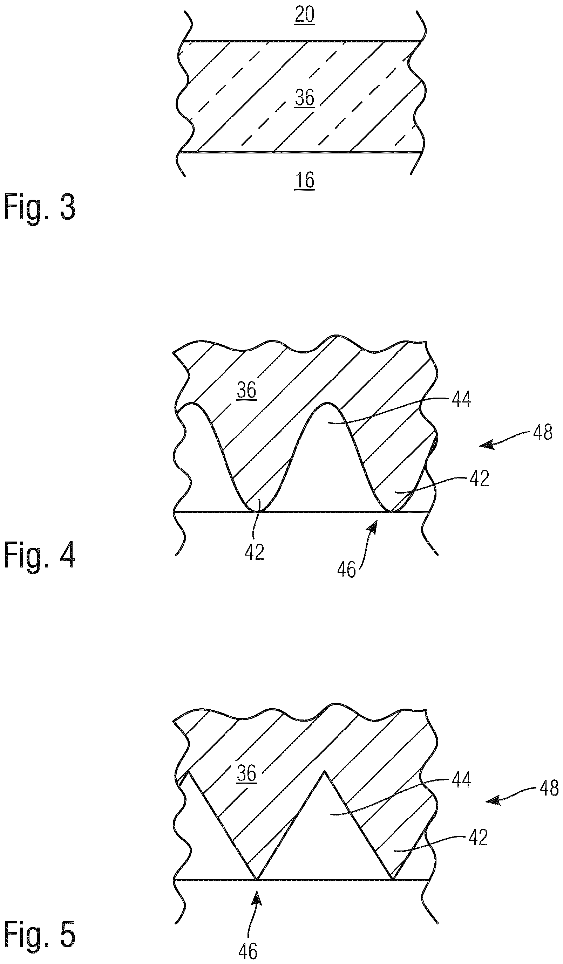

In Fig. 3 ist eine Ausschnittsvergrößerung entsprechend dem in Fig. 2 dargestellten Kreis ersichtlich. Bei der Ausführungsform gemäß Fig. 3 kommt ein anderes erfindungsgemäßes Gleitelement 36 zum Einsatz. Die Folie 20 ist bei der Ausführungsform gemäß Fig. 3 mit Para-Xylylene beschichtet. Auch hier handelt es sich um eine atomare Abscheidung. Die Oberfläche der Para-Xylylene-Beschichtung ist homogener und glatt und bietet einen geringen Gleitreibungskoeffizienten gegenüber dem aus Polysulfon bestehenden Schaft 16.

Der Gleitreibungskoeffizient beträgt mit gewissen Schwankungen im Durchschnitt zwischen 0,9 und 1,2.

Eine demgegenüber weiter modifizierte Ausführungsform eines erfindungsgemäßen Lippen/Wangen-Halters 12 ist aus Fig. 4 ersichtlich. Bei dieser Ausführungsform sind Mikrostrukturen auf oder an der Oberfläche des Elastomers der Folie 20 erzeugt. Diese bestehen aus Bergen 42 und Tälern 44, die einander abwechseln. Die Berge 42 springen zum Schaft 16 hin vor und die Täler 44 springen von diesem zurück.

Lediglich die Berge bilden Kontaktbereiche 46. Die Kontaktbereiche 46 des Gleitelements 36 verbleiben bei Dehnung der Folie 20 unverändert oder im Wesentlichen, also zu mehr als 80%, unverändert, und die Dehnung vergrößert die Abstände zwischen den Kontaktbereichen 46.

Die Berge 42 sind gleichmäßig über die Oberfläche des Gleitelements 36 verteilt. Die Größe der Berge 42, also die Dicke der Mikrostruktur 48 kann in beliebiger geeigneter Weise gewählt werden, beispielsweise zwischen dem atomaren Bereich und wenigen Mikrometern. Die Mikrostruktur 48 kann in Negativform in die Oberfläche einer Spritzgussform eingearbeitet werden, und durch das Spritzgießen entsteht dann automatisch die Mikrostruktur 48 des Gleitelements 36.

Eine demgegenüber weiter modifizierte Ausführungsform eines erfindungsgemäßen Lippen/Wangen-Halters 12 ist aus Fig. 5 ersichtlich. Bei dieser Ausführungsform sind Mikrostrukturen 48 in Form von Pyramiden ausgebildet. Diese weisen spitze Berge 42 und Täler 44 auf, die ebenfalls spitz zulaufen. Die Ausgestaltung der Täler spielt jedoch keine Rolle hinsichtlich des Haftreibungskoeffizienten.

Diese Ausgestaltung ergibt noch geringere Reibungskoeffizienten als die gemäß Fig. 4 mit den balligen Bergen 42.

Es wurden Versuche zur Haftreibung und zur Gleitreibung mit Polysulfon als Reibungspartner durchgeführt. Polysulfon wurde gewählt, da der Schaft 16 von Intraoralscannern 10 häufig aus Polysulfon besteht. Der Anpressdruck wurde zwischen 1 und 5 N variiert. Die Bewegungsgeschwindigkeit bei der Gleitreibungsmessung betrug etwa 6mm/s.

Ferner wurde die Mikrostruktur-Größe geändert zwischen den Rastermassen 50µm und 200 µm und der Pyramidenhöhe zwischen 24 µm und 87 µm.

Der Reibungskoeffizient wurde je nach Anpressdruck im Vergleich mit einem Elastomerstreifen zwischen 22 und 25 %, oder bei einer anderen Versuchsanordnung um 37%, reduziert, und zwar bei der maximalen geprüften Mikrostruktur-Größe.

Eine erheblich größere Schwankungsbreite ergab sich bei einer Vergleichsmessung mit einer Siloxan-Beschichtung anstelle der Mikrostruktur 48. Die Reduktion des Reibungskoeffizienten schwankte dann je nach Anspressdruck zwischen 21 und 49%.

Wesentlich ist, dass der Unterschied zwischen Haftreibungskoeffizient und Gleitreibungskoeffizient bei der erfindungsgemäßen Ausgestaltungen je geringer als bei einer unbeschichteten und unbehandelten Elastomerfolie war.

Dieser Effekt reicht aus, um den Stick-Slip-Effekt zu vermeiden. Die erfindungsgemäßen Ausführungsformen haben den besonderen Vorteil, dass je ein vergleichsweise geringer Unterschied zwischen Haftreibungskoeffizient und Gleitreibungskoeffizient besteht

Messungen haben ergeben, dass eine unbeschichtete und unbehandelte Elastomerfolie wie ein Standard-Optragate einen Hafttreibungskoeffizient von etwa 3,5 bis 4 und Gleitreibungskoeffizient von etwa 2,5 hat.

## Patentansprüche

1. Lippen/Wangen-Halter, mit einer Folie aus einem Elastomer, einem Lippenring und einem Vestibulärring, welche Folie sich zwischen dem Lippenring und dem Vestibulärring erstreckt, von diesen aufgespannt gehalten ist und zwischen diesen einen schlauchförmigen Abschnitt bildet, **dadurch gekennzeichnet, dass** die Folie (20) an der zu einem Schaft hin weisenden Innenseite (32) ihres schlauchförmigen Abschnitts mit einem, insbesondere die Oberfläche der Folie (20) dort verändernden, durch eine Oberflächenbehandlung erzeugtes, fest mit der Folie (20) verbundenes Gleitelement (36), vorgefertigt ausgestattet ist.

2. Lippen/Wangen-Halter nach Anspruch 1, **dadurch gekennzeichnet, dass** die Größenangabe des Gleitreibungskoeffizienten µ_{G} des Gleiteelements (36) sich auf Materialpaarung Folie/formstabiler Kunststoff oder Folie/Metall bezieht.

3. Lippen/Wangen-Halter nach Anspruch 1 oder 2, **dadurch gekennzeichnet, dass** das Gleitelement (36) zur Bereitstellung des Gleitreibungskoeffizienten eine mit der Folie (20) fest verbundene Beschichtung aus SiO₂ aufweist.

4. Lippen/Wangen-Halter nach einem der vorhergehenden Ansprüche,
**dadurch gekennzeichnet, dass** das Gleitelement (36) durch eine Kombination aus atomarer Abscheidung und Oberflächenmodifikation, oder auch Oberflächenstrukturierung,erzeugt ist.

5. Lippen/Wangen-Halter nach Anspruch 1 oder 2, **dadurch gekennzeichnet, dass** das Gleitelement (36) zur Bereitstellung des Gleitreibungskoeffizienten eine mit der Folie (20) fest verbundene Beschichtung aus Para-Xylylene aufweist.

6. Lippen/Wangen-Halter nach Anspruch 1 oder 2, **dadurch gekennzeichnet, dass** das Gleitelement (36) zur Bereitstellung des Gleitreibungskoeffizienten eine per Spritzgießen erzeugte Mikrostruktur (48) mit Bergen (42) (vorspringenden Bereichen) und Tälern (44) (rückspringenden Bereichen) aufweist, deren Berge (42) insbesondere härter sind als das Elastomer der Folie (20) und insbesondere weniger als 30 % der Fläche des Gleitelements (20) einnehmen.

7. Lippen/Wangen-Halter nach einem der vorhergehenden Ansprüche,
**dadurch gekennzeichnet, dass** das Gleitelement (36) Kontaktbereiche (46) aufweist, die je eine Größe zwischen dem atomaren Bereich und wenigen µm aufweisen und gleichmäßig über das Gleitelement (36) verteilt sind.

8. Lippen/Wangen-Halter nach einem der Ansprüche 6 oder 7, **dadurch gekennzeichnet, dass** Berge (42), vorspringende Bereiche oder abstehende Bereiche der Mikrostruktur (48) die Kontaktbereiche (46) bilden.

9. Lippen/Wangen-Halter nach einem der vorhergehenden Ansprüche,
**dadurch gekennzeichnet, dass** Kontaktbereiche (46) des Gleitelements bei Dehnung der Folie (20) unverändert oder im Wesentlichen, also zu mehr als 80%, unverändert, verbleiben und die Dehnung die Abstände zwischen den Kontaktbereichen (46) vergrößert.

10. Lippen/Wangen-Halter nach einem der vorhergehenden Ansprüche,
**dadurch gekennzeichnet, dass** das Gleitelement (36) sich über die gesamte Innenseite (32) des schlauchförmigen Abschnitts der Folie (20) erstreckt.

11. Lippen/Wangen-Halter nach einem der Ansprüche 1 bis 11, **dadurch gekennzeichnet, dass** die Folie (20) in aufgespanntem Zustand zwischen dem Lippenring und dem Vestibulärring eine Einschnürung aufweist und dass das Gleitelement sich über die Innenseite des schlauchförmigen Abschnitts der Folie lediglich im Bereich der Einschnürung, also von den Ringen beabstandet, erstreckt.

12. Lippen/Wangen-Halter nach einem der vorhergehenden Ansprüche, **dadurch gekennzeichnet, dass** das Gleitelement (36) eine Stärke zwischen 2 µm und 200 µm, bevorzugt 5 bis 150 µm, aufweist.

13. Lippen/Wangen-Halter nach einem der vorhergehenden Ansprüche, **dadurch gekennzeichnet, dass** das Gleitelement (36) auf die Folie (20) aufgebracht und mit dieser fest verbunden ist.

14. Lippen/Wangen-Halter nach einem der vorhergehenden Ansprüche, **dadurch gekennzeichnet, dass** das Gleitelement (36) eine geringere Elastizität als das Elastomer der Folie (20) hat und härter als dieses ist.

15. Lippen/Wangen-Halter nach einem der vorhergehenden Ansprüche, **dadurch gekennzeichnet, dass** das Gleitelement (36) wasserabweisend ausgebildet ist.

16. System aus einem Intraoralscanner und einem Lippen/Wangen-Halter, welcher Intraoralscanner ein Außengehäuse aus Kunststoff und/oder Metall aufweist und welcher Lippen/Wangen-Halter eine Folie aus einem Elastomer, einen Lippenring und einen Vestibulärring aufweist, die sich zwischen dem Lippenring und dem Vestibulärring erstreckt, von diesen aufgespannt gehalten ist und zwischen diesen einen schlauchförmigen Abschnitt bildet, **dadurch gekennzeichnet, dass** die Folie (20) an der zu einem Schaft hin weisenden Innenseite (32) ihres schlauchförmigen Abschnitts mit einem, insbesondere die Oberfläche der Folie (20) dort verändernden, durch eine Oberflächenbehandlung erzeugtes, fest mit der Folie (20) verbundenes Gleitelement (36) gegenüber dem Schaft (16) des Intraoralscanners (10), vorgefertigt ausgestattet ist.

## Claims

1. A lip/cheek retainer comprising a film made of elastomer, a lip ring and a vestibular ring, said film extending between the lip ring and the vestibular ring, being held stretched by them and forming a tubular section between them, **characterized in that** the film (20) is provided in a prefabricated manner on the inner side (32) of its tubular section pointing to a shaft with a sliding element (36), firmly connected to the film (20), produced by a surface treatment which in particular alters the surface of the film (20) thereat.

2. The lip/cheek retainer according to claim 1, **characterized in that** the size specification of the sliding friction coefficient µ_{G} of the sliding element (36) refers to the material pairing film/shape-retaining plastic or film/metal.

3. The lip/cheek retainer according to claim 1 or 2, **characterized in that** the sliding element (36) has a coating of SiO₂ firmly connected to the film (20) to provide the sliding friction coefficient.

4. The lip/cheek retainer according to one of the preceding claims, **characterized in that** the sliding element (36) is produced by a combination of atomic deposition and surface modification, or surface texturing.

5. The lip/cheek retainer according to claim 1 or 2, **characterized in that** the sliding element (36) comprises a coating of para-xylylene firmly connected to the film (20) to provide the sliding friction coefficient

6. The lip/cheek retainer according to claim 1 or 2, **characterized in that**, to provide the sliding friction coefficient, the sliding element (36) has a microstructure (48) produced by injection moulding and having peaks (42) (projecting regions) and valleys (44) (recessed regions), the peaks (42) of which are in particular harder than the elastomer of the film (20) and in particular occupy less than 30% of the surface of the sliding element (20).

7. The lip/cheek retainer according to one of the preceding claims, **characterized in that** the sliding element (36) has contact regions (46), the size of each of which lies between the atomic range and a few um and which are uniformly distributed over the sliding element (36).

8. The lip/cheek retainer according to one of claims 6 or 7, **characterized in that** peaks (42), projecting regions or protruding regions of the microstructure (48) form the contact regions (46).

9. The lip/cheek retainer according to one of the preceding claims, **characterized in that** contact regions (46) of the sliding element remain unchanged or substantially unchanged, i.e. more than 80%, when the film (20) is stretched, and the stretching increases the distances between the contact regions (46) .

10. The lip/cheek retainer according to one of the preceding claims, **characterized in that** the sliding element (36) extends over the entire inner side (32) of the tubular section of the film (20).

11. The lip/cheek retainer according to one of the claims 1 to 11, **characterized in that** the film (20) in the stretched state has a constriction between the lip ring and the vestibular ring and that the sliding element extends over the inner side of the tubular section of the film only in the region of the constriction, i.e. at a distance from the rings.

12. The lip/cheek retainer according to one of the preceding claims, **characterized in that** the sliding element (36) has a thickness of between 2 um and 200 um, preferably 5 to 150 µm.

13. The lip/cheek retainer according to one of the preceding claims, **characterized in that** the sliding element (36) is applied to and firmly connected to the film (20).

14. The lip/cheek retainer according to one of the preceding claims, **characterized in that** the sliding element (36) has a lower elasticity than the elastomer of the film (20) and is harder than the latter.

15. The lip/cheek retainer according to one of the preceding claims, **characterized in that** the sliding element (36) is configured to be water-repellent.

16. A system comprising an intraoral scanner and a lip/cheek retainer, said intraoral scanner having an outer housing made of plastic and/or metal and said lip/cheek retainer having a lip ring, a vestibular ring and a film made of elastomer which extends between the lip ring and the vestibular ring, is held stretched by these and forms a tubular section between them, **characterized in that** the film (20) is provided in a prefabricated manner on the inner side (32) of its tubular section pointing to a shaft with a sliding element (36), with respect to the shaft (16) of the intraoral scanner (10), which is firmly connected to the film (20) and produced by a surface treatment which in particular alters the surface of the film (20) thereat.

## Revendications

1. Support de lèvres/joues, comprenant une feuille en élastomère, un anneau labial et un anneau vestibulaire, où la feuille s'étend entre l'anneau labial et l'anneau vestibulaire, est maintenue tendue par ceux-ci et forme entre eux une section tubulaire, **caractérisé en ce que** la feuille (20) est équipée, sur la face intérieure (32) de sa section tubulaire orientée vers une tige, d'un élément de glissement (36), en particulier modifiant la surface de la feuille (20) à cet endroit, produit par un traitement de surface et relié de manière fixe à la feuille (20).

2. Support de lèvres/joues selon la revendication 1, selon la revendication 1, **caractérisé en ce que** l'indication de taille du coefficient de frottement de glissement µG de l'élément de glissement (36) se rapporte à une paire de matériaux feuille/plastique indéformable ou feuille/métal.

3. Support de lèvres/joues selon la revendication 1 ou 2, **caractérisé en ce que** l'élément de glissement (36) présente un revêtement en SiO₂ solidaire de la feuille (20) pour fournir le coefficient de frottement de glissement.

4. Support de lèvres/joues selon l'une des revendications précédentes, **caractérisé en ce que** l'élément de glissement (36) est produit par une combinaison de dépôt atomique et de modification de surface, ou également de la structuration de surface.

5. Support de lèvres/joues selon la revendication 1 ou 2, **caractérisé en ce que** l'élément de glissement (36) présente un revêtement en para-xylylène solidaire de la feuille (20) pour fournir le coefficient de frottement de glissement.

6. Support de lèvres/joues selon la revendication 1 ou 2, **caractérisé en ce que** l'élément de glissement (36) présente, pour fournir le coefficient de frottement de glissement, une microstructure (48) produite par moulage par injection avec des crêtes (42) (zones en saillie) et des creux (44) (zones en retrait), dont les crêtes (42) sont en particulier plus dures que l'élastomère de la feuille (20) et occupent en particulier moins de 30 % de la surface de l'élément de glissement (20).

7. Support de lèvres/joues selon l'une des revendications précédentes, **caractérisé en ce que** l'élément de glissement (36) comprend des zones de contact (46) qui ont chacune une taille comprise entre la zone atomique et quelques pm et sont réparties uniformément sur l'élément de glissement (36).

8. Support de lèvres/joues selon l'une des revendications 6 ou 7, **caractérisé en ce que caractérisée en ce que** des crêtes (42), des zones en saillie ou des zones qui dépassent la microstructure (48) forment les zones de contact (46).

9. Support de lèvres/joues selon l'une des revendications précédentes, **caractérisé en ce que** des zones de contact (46) de l'élément coulissant restent inchangées ou sensiblement inchangées, c'est-à-dire à plus de 80%, lorsque la feuille (20) est étiré, et l'étirement augmente les distances entre les zones de contact (46).

10. Support de lèvres/joues selon l'une des revendications précédentes, **caractérisé en ce que** l'élément de glissement (36) s'étend sur toute la face intérieure (32) de la partie tubulaire de la feuille (20).

11. Support pour lèvres/joues selon l'une des revendications 1 à 11, **caractérisé en ce que** la feuille (20), à l'état tendu, présente un rétrécissement entre l'anneau labial et l'anneau vestibulaire, et **en ce que** l'élément de glissement s'étend sur la face intérieure de la section tubulaire de la feuille uniquement dans la zone du rétrécissement, c'est-à-dire à distance des anneaux.

12. Support de lèvres/joues selon l'une des revendications précédentes, **caractérisé en ce que** l'élément de glissement (36) présente une épaisseur comprise entre 2 pm et 200 pm, de préférence entre 5 et 150 pm.

13. Support de lèvres/joues selon l'une des revendications précédentes, **caractérisé en ce que** l'élément de glissement (36) est appliqué sur la feuille (20) et est relié de manière fixe à celle-ci.

14. Support de lèvres/joues selon l'une des revendications précédentes, **caractérisé en ce que** l'élément de glissement (36) a une élasticité inférieure à celle de l'élastomère du film (20) et est plus dur que celui-ci.

15. Support de lèvres/joues selon l'une des revendications précédentes, **caractérisé en ce que** l'élément coulissant (36) est configuré de manière à repousser l'eau.

16. Système composé d'un scanner infra-oral et d'un support de lèvres/joues, lequel scanner infra-oral présente un boîtier extérieur en matière plastique et/ou en métal et lequel support de lèvres/joues présente une feuille en élastomère, un anneau labial et un anneau vestibulaire, qui s'étend entre l'anneau labial et l'anneau vestibulaire, est maintenue tendue par ceux-ci et forme entre eux une section tubulaire, **caractérisé en ce que** la feuille (20) est préalablement équipée, sur la face intérieure (32) de sa section tubulaire orientée vers une tige, d'un élément de glissement (36), en particulier modifiant à cet endroit la surface de la feuille (20), produit par un traitement de surface et relié solidement à la feuille (20) par rapport à la tige (16) du scanner infra-oral (10).
